# EUROPÄISCHE PATENTANMELDUNG

(11) **EP 1 580 550 A2**
(43) Veröffentlichungstag der Anmeldung: **28.09.2005**
(21) Anmeldenummer: 05100538.7
(22) Anmeldetag: 27.01.2005
(51) Int. Cl.: G01N 33/30, F01M 11/12, G01N 11/06

(54) **Verfahren zum Ermitteln einer für die Viskosität eines Brennkraftmaschinenöls charakteristischen Grösse**

(30) Priorität: 26.03.2004 DE 102004015003
(71) Anmelder: SIEMENS AKTIENGESELLSCHAFT, 80333 München (DE)
(72) Erfinder: Gilch, Markus, 85419, Mauern (DE); Heinrich, Stephan, 93053, Regensburg (DE); Ott, Andreas, 93128, Steinsberg (DE)

(57) **Zusammenfassung**

Eine Brennkraftmaschine hat einen Ölstandssensor, der in einer Ölwanne (12) angeordnet ist und der ein Messrohr (30) hat, das über mindesten eine Ausnehmung (32, 34) mit dem das Messrohr (30) umgebenden Öl kommuniziert. Es wird geprüft, ob vorgegebenen Betriebsbedingungen (BB) vorliegen. Wenn die vorgegebenen Betriebsbedingungen (BB) vorliegen, wird eine zeitliche Veränderlichkeit (GRAD_OIL) des Ölstands (OIL_H) in dem Messrohr (30) ermittelt. Abhängig von der zeitlichen Veränderlichkeit (GRAD_OIL) des Ölstands (OIL_H) wird eine Größe ermittelt, die charakteristisch ist für die Viskosität des Öls.

## Beschreibung

Die Erfindung betrifft ein Verfahren zum Ermitteln einer Größe, die charakteristisch ist für die Viskosität eines Öls einer Brennkraftmaschine.

Das Öl einer Brennkraftmaschine wird eingesetzt zum Schmieren mechanisch beweglicher Teile der Brennkraftmaschine, wie zum Beispiel der Kolben und der Zylinderwände. Für einen hohen Wirkungsgrad der Brennkraftmaschine und zum Gewährleisten eines geringen Verschleißes an der Brennkraftmaschine ist es wesentlich, dass sich die Viskosität des Öls innerhalb eines vorgebbaren Bereichs befindet. Die Viskosität des Öls der Brennkraftmaschine kann jedoch während des Betriebs deutlichen Änderungen unterliegen. So werden beispielsweise durch Vor- und Nacheinspritzungen von Kraftstoff, zum Beispiel Diesel, die Schadstoff-Rohemissionen der Brennkraftmaschine verringert. Andererseits wird jedoch durch derartige Vor- oder Nacheinspritzungen gegebenenfalls Öl von den Wänden der Zylinder der Brennkraftmaschine abgewaschen und es gelangt somit auch Kraftstoff in einen Ölkreislauf der Brennkraftmaschine. Kraftstoff kann ferner in den Ölkreislauf der Brennkraftmaschine gelangen durch Lecks an Einspritzventilen oder Kraftstoffpumpen. Dadurch verschlechtern sich die Schmiereigenschaften des Öls. Ferner kann es während des Betriebs der Brennkraftmaschine auch zu einer Bildung von Schwarzschlamm kommen, der ungünstig ist für den Betrieb der Brennkraftmaschine.

Durch ein Erfassen oder Ermitteln der Viskosität, oder einer Größe, die die Viskosität charakterisiert, kann so zum Beispiel durch entsprechende Auswertung ein Fahrer eines Kraftfahrzeugs, in dem die Brennkraftmaschine angeordnet ist, darauf hingewiesen werden, dass das Öl der Brennkraftmaschine gewechselt werden muss.

Zu diesem Zweck sind Sensoren bekannt, die die Viskosität eines Fluids erfassen. Aus der DE 101 12 433 A1 ist eine Messanordnung für einen Viskositätsmessung von Flüssigkeiten bekannt. Die Anordnung umfasst einen Behälter, der sich vollständig in der zu messenden Flüssigkeit befindet und über Öffnungen mit der Flüssigkeit kommuniziert. In dem Behälter ist ein scheibenförmiges piezoelektrisches Element angeordnet. Das Piezoelement hat zwei elektrische Kontaktstellen, die mit elektrischen Zuführleitungen kontaktiert sind, die als Federelemente ausgebildet sind. Das Piezoelement ist durch entsprechende elektrische Ansteuerungen zu Scherschwingungen anregbar. Die Viskosität des Fluids, das sich in dem Behälter befindet wird anhand einer geänderten Resonanzfrequenz des Piezoelements ermittelt. Eine derartige Messanordnung ist jedoch teuer und zeigt auch eine Anfälligkeit gegenüber Oberflächenschichten, die sich im Laufe der Zeit an dem Piezoelement ablagern können und so zu Fehlmessungen führen können.

Die Aufgabe der Erfindung ist es, ein Verfahren zu schaffen, das ein einfaches Ermitteln einer Größe ermöglicht, die charakteristisch ist für die Viskosität eines Öls einer Brennkraftmaschine.

Die Aufgabe wird gelöst durch die Merkmale des unabhängigen Patentanspruchs. Vorteilhafte Ausgestaltungen der Erfindung sind in den Unteransprüchen gekennzeichnet.

Die Erfindung zeichnet sich aus durch ein Verfahren zum Ermitteln einer Größe, die charakteristisch ist für die Viskosität eines Öls einer Brennkraftmaschine mit einem Ölstandssensor, der in einer Ölwanne angeordnet ist und der ein Messrohr hat, das über mindestens eine Ausnehmung mit dem das Messrohr umgebenden Öl kommuniziert. Bei dem Verfahren wird geprüft, ob vorgegebene Betriebsbedingungen vorliegen. Wenn die vorgegebenen Betriebsbedingungen vorliegen, wird eine zeitliche Veränderlichkeit des Ölstands in dem Messrohr ermittelt. Die zeitliche Veränderlichkeit des Ölstands in dem Messrohr kann auch als Gradient des Ölstands in dem Messrohr bezeichnet werden. Die zeitliche Veränderlichkeit des Ölstandes im Messrohr ist direkt proportional zur Abflussgeschwindigkeit des Öls aus dem Messrohr durch die mindestens eine Ausnehmung. Abhängig von der zeitlichen Veränderlichkeit des Ölstands wird die Größe ermittelt, die charakteristisch ist für die Viskosität des Öls. Auf diese Weise wird einfach der ohnehin häufig vorhandene Ölstandssensor genutzt, um die Größe zu ermitteln, die charakteristisch ist für die Viskosität des Öls. Ölstandssensoren finden zunehmend Einsatz in Brennkraftmaschinen, um zum einen den Fahrer darauf hinzuweisen, dass entweder der Ölstand zu niedrig oder zu hoch ist, und andererseits um den Ölstand für den Fahrzeughersteller zu protokollieren, gegebenenfalls im Hinblick auf Gewährleistungsansprüche.

Durch das erfindungsgemäße Verfahren ist es möglich, die Größe, die charakteristisch ist für die Viskosität des Öls, ohne einen zusätzlichen Sensor zu ermitteln. Die Erfindung nutzt dabei die Erkenntnis, dass die zeitliche Veränderlichkeit des Ölstands in dem Messrohr des Ölstandssensors charakteristisch ist für die Fließgeschwindigkeit des Öls und somit charakteristisch ist für die Viskosität des Öls.

In einer vorteilhaften Ausgestaltung der Erfindung hängen die Betriebsbedingungen ab von einer Stillstandszeitdauer der Brennkraftmaschine. Dem liegt die Erkenntnis zugrunde, dass die Stillstandszeitdauer maßgeblich ist für die Präzision des Bestimmens der Größe, die charakteristisch ist für die Viskosität des Öls. Je länger die Stillstandszeit ist, desto weniger Öl befindet sich außerhalb der Ölwanne in dem Ölkreislauf der Brennkraftmaschine. Der Verlauf des Ölstandes ist dann bei einem erneuten Start der Brennkraftmaschine sehr gut reproduzierbar, womit sehr gute Messbedingungen vorliegen. Ferner ist auch die Höhe des Ölstands abhängig von der Stillstandszeitdauer, was sich vorteilhaft auf die Messung auswirkt.

In einer weiteren vorteilhaften Ausgestaltung der Erfindung hängen die vorgegebenen Betriebsbedingungen ab von einer erfassten Öltemperatur und/oder einer Umgebungstemperatur und/oder einer Kühlmitteltemperatur. Auf diese Weise kann das Ermitteln der Größe, die charakteristisch ist für die Viskosität des Öls, sehr präzise erfolgen, da die Viskosität sehr stark abhängt von der Temperatur des Öls in dem Messrohr des Ölstandssensors. Somit kann die Güte des Bestimmens der Größe, die charakteristisch ist für die Viskosität des Öls, auf diese Weise deutlich verbessert werden.

In diesem Zusammenhang ist vorteilhaft, wenn die vorgegebenen Betriebsbedingungen nur dann erfüllt sein können, wenn die erfasste Öltemperatur und/oder eine Umgebungstemperatur der Brennkraftmaschine und/oder eine Kühlmitteltemperatur sich weniger als ein vorgegebener Schwellenwert ändern. Bei geeigneter Wahl des vorgegebenen Schwellenwertes, der für die erfasste Öltemperatur und/oder die Umgebungstemperatur und/oder die Kühlmitteltemperatur unterschiedlich sein kann, ändert sich somit während des Erfassens der Größen, aus denen die zeitliche Veränderlichkeit des Ölstands ermittelt wird, die Temperatur des Öls in dem Messrohr nur unwesentlich. Damit ist dann auch sichergestellt, dass sich die Viskosität des Öls in dem Messrohr während des Ermittelns der Größen, die zum Ermitteln der zeitlichen Veränderlichkeit benötigt werden, nur unwesentlich ändert und damit eine präzise Ermittlung der Größe gewährleistet ist, die charakteristisch ist für die Viskosität des Öls.

In einer weiteren vorteilhaften Ausgestaltung der Erfindung hängen die vorgegebenen Betriebsbedingungen ab von einer Drehzahl einer Kurbelwelle einer Brennkraftmaschine und/oder einer Fahrzeuggeschwindigkeit eines Kraftfahrzeugs, in dem die Brennkraftmaschine angeordnet ist, und/oder einer Beschleunigung des Kraftfahrzeugs. Auf diese Weise kann einfach die Güte des Bestimmens der Größe, die charakteristisch ist für die Viskosität des Öls, erhöht werden, da sich die Drehzahl der Kurbelwelle, die Fahrgeschwindigkeit des Kraftfahrzeugs und die Beschleunigung des Kfzs stark auf die Güte des Bestimmens der Größe auswirken.

In einer weiteren vorteilhaften Ausgestaltung der Erfindung hängen die vorgegebenen Betriebsbedingungen davon ab, ob die zeitliche Veränderlichkeit des Ölstands im Wesentlichen monoton fällt. Auf diese Weise kann auch eine hohe Güte des Bestimmens der Größe gewährleistet werden, die charakteristisch ist für die Viskosität des Öls.

Es ist ferner vorteilhaft, wenn die zeitliche Veränderlichkeit des Ölstands während des Vorliegens der vorgegebenen Betriebsbedingungen gemittelt wird. Auf diese Weise kann sichergestellt werden, dass einzelne Messfehler beim Bestimmen der zeitlichen Veränderlichkeit sich weniger stark auswirken und auf diese Weise kann auch eine einfache Datenreduktion erreicht werden.

In einer weiteren vorteilhaften Ausgestaltung der Erfindung wird die zeitliche Veränderlichkeit des Ölstands verglichen mit einer vorgegebenen zeitlichen Veränderlichkeit des Ölstands und abhängig von dem Vergleich der zeitlichen Veränderlichkeiten wird die Größe ermittelt, die charakteristisch ist für die Viskosität des Öls. Auf diese Weise kann die Größe, die charakteristisch ist für die Viskosität einfach und präzise ermittelt werden, insbesondere da die vorgegebene zeitliche Veränderlichkeit einfach durch Versuche an einem Motorprüfstand oder Simulationen ermittelt werden kann.

In einer weiteren vorteilhaften Ausgestaltung der Erfindung wird die Größe, die charakteristisch ist für die Viskosität, abhängig davon ermittelt für welche Zeitdauer die Betriebsbedingungen durchgehend vorliegen. Dem liegt die Erkenntnis zugrunde, dass die Güte des Ermittelns der Größe, die charakteristisch ist für die Viskosität abhängig ist von der Zeitdauer für welche die Betriebsbedingungen durchgehend vorliegen. So kann einfach die Güte beim Ermitteln der Größe, die charakteristisch ist für die Viskosität, erhöht werden.

In einer weiteren vorteilhaften Ausgestaltung der Erfindung ist die Größe, die charakteristisch ist für die Viskosität, die Viskosität und die Viskosität wird abhängig von der zeitlichen Veränderlichkeit des Ölstands und der erfassten Öltemperatur und/oder einer Umgebungstemperatur der Brennkraftmaschine und/oder der Kühlmitteltemperatur ermittelt.

In einer weiteren vorteilhaften Ausgestaltung der Erfindung ist die Größe, die charakteristisch ist für die Viskosität des Öls, die zeitliche Veränderlichkeit des Ölstands. Dies hat den Vorteil, dass dieser sehr einfach zu ermitteln ist.

In einer weiteren vorteilhaften Ausgestaltung der Erfindung ist die Größe, die charakteristisch ist für die Viskosität, eine Viskositätsänderung. Dies hat den Vorteil, dass die Viskositätsänderung relativ einfach zu ermitteln ist und die Viskositätsänderung ausreicht zum Beurteilen, ob ein Wechsel des Öls in der Brennkraftmaschine notwendig ist.

Ausführungsbeispiele der Erfindung sind im Folgenden anhand der schematischen Zeichnungen erläutert. Es zeigen:
- Figur 1: eine Brennkraftmaschine,

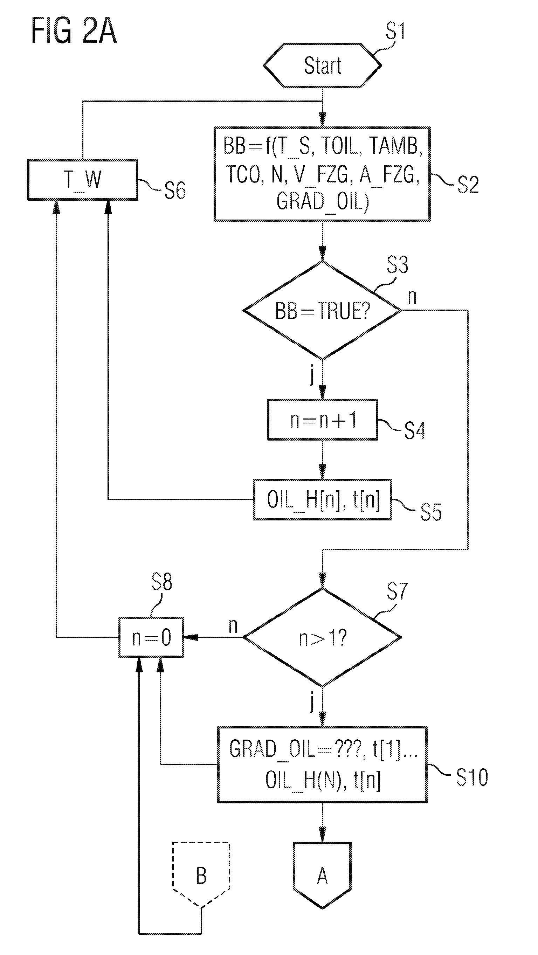

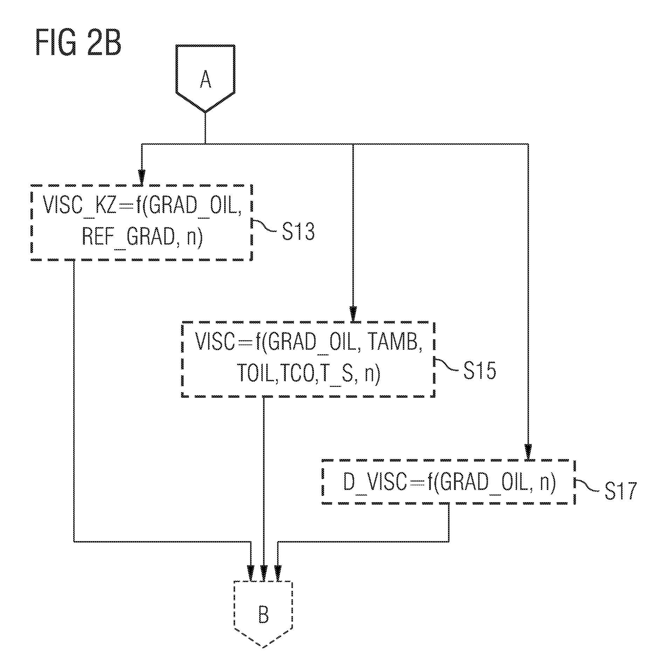

- Figur 2a und 2b: ein Ablaufdiagramm eines Programms zum Ermitteln einer Größe, die charakteristisch ist für die Viskosität des Öls,
- Figur 3: einen ersten Signalverlauf verschiedener Messgrößen und
- Figur 4: einen zweiten Signalverlauf verschiedener Messgrößen.

Elemente gleicher Konstruktion oder Funktion sind figuren-übergreifend mit den gleichen Bezugszeichen gekennzeichnet.

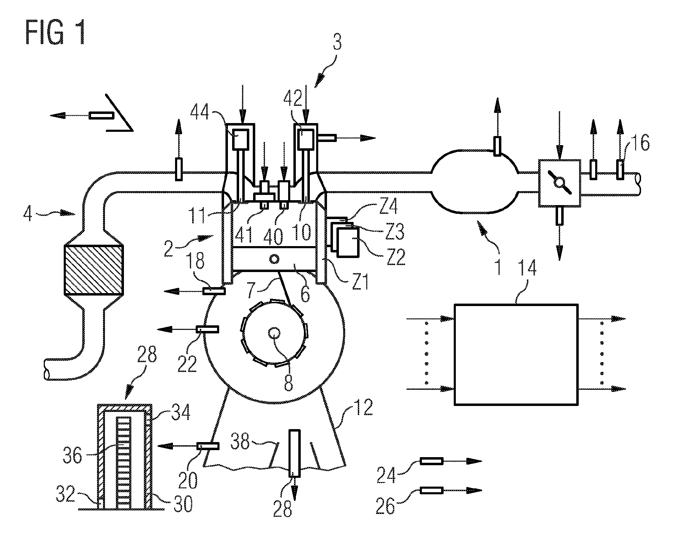

Eine Brennkraftmaschine (Figur 1) hat einen Ansaugtrakt 1, einen Motorblock 2, einen Zylinderkopf 3 und einen Abgastrakt 4. In dem Motorblock 2 sind Zylinder Z1 bis Z4 ausgebildet, denen jeweils ein Kolben 6 zugeordnet ist, der über eine Pleuelstange 7 mit einer Kurbelwelle 8 gekoppelt ist. In dem Zylinderkopf 3 ist ein Gaseinlassventil 10 angeordnet, abhängig von dessen Stellung der jeweilige Zylinder Z1-Z4 mit dem Ansaugtrakt 1 kommuniziert. Ferner ist in dem Zylinderkopf ein Gasauslassventil 11 angeordnet, abhängig von dessen Stellung der jeweilige Zylinder Z1 - Z4 mit dem Abgastrakt 4 kommuniziert.

Dem Motorblock 2 ist ferner eine Ölwanne 12 zugeordnet, die ein Reservoir für Öl eines Ölkreislaufs der Brennkraftmaschine bildet. Mittels des Ölkreislaufs wird beispielsweise der Kolben 6 geschmiert, so dass die Reibung zwischen der Innenwand des jeweiligen Zylinders Z1 bis Z4 und dem Kolben 6 möglichst gering ist. Mittels des Öls werden beispielsweise auch Führungen des Gaseinlassventils 10 und des Gasauslassventils 11 geschmiert. Das in der Ölwanne 12 befindliche Öl wird mittels einer nicht dargestellten Ölpumpe in dem Ölkreislauf bewegt.

Der Brennkraftmaschine ist eine Steuereinrichtung 14 zugeordnet, die auch als Vorrichtung zum Steuern der Brennkraftmaschine bezeichnet werden kann. Der Steuereinrichtung 14 sind Sensoren zugeordnet, die Messgrößen erfassen. Ferner sind der Steuereinrichtung 14 Stellglieder zugeordnet, die mittels entsprechender Stellantriebe angetrieben werden. Abhängig von den von den Sensoren erfassten Messgrößen ermittelt die Steuereinrichtung 14 Stellgrößen zum Ansteuern der Antriebe der Stellglieder.

Die Sensoren sind ausgebildet als ein erster Temperatursensor 16, der eine Umgebungstemperatur TAMB erfasst, ein zweiter Temperatursensor 18, der eine Kühlmitteltemperatur TCO erfasst, ein dritter Temperatursensor 20, der eine Öltemperatur TOIL erfasst, ein Drehzahlsensor 22, welche eine Drehzahl N der Kurbelwelle 8 erfasst, ein Fahrgeschwindigkeitssensor 24, welcher eine Fahrzeuggeschwindigkeit V_FZG erfasst, ein Beschleunigungssensor 26, welcher eine Fahrzeugbeschleunigung A_FZG erfasst und ein Ölstandssensor 28, welcher einen Ölstand OIL_H erfasst. Je nach Ausführungsform der Steuereinrichtung 14 können mehr oder auch weniger als die genannten Sensoren vorhanden sein. Insbesondere kann beispielsweise die Fahrgeschwindigkeit V_FZG abhängig von der Drehzahl N und einer Getriebeübersetzung ermittelt werden. Das gleiche gilt für die Fahrzeugbeschleunigung A_FZG.

Die Stellglieder sind beispielsweise ein Einspritzventil 40, eine Zündkerze 41 oder die Gaseinlassventile 10 und die Gasauslassventile 11, denen dann jeweils ein Ventilantrieb 42, 44 zugeordnet ist.

Der Ölstandssensor 28 ist bevorzugt im Bereich eines Bodens der Ölwanne 12 angeordnet. Er hat ein Messrohr 30 mit einer ersten Ausnehmung 32, die bevorzugt in der Nähe des Bodens der Ölwanne 12 ausgebildet ist, und eine zweite Ausnehmung 34, die möglichst weit von dem Boden der Ölwanne 12 beabstandet ausgebildet ist. In dem Messrohr 30 ist ferner ein Sensorelement 36 angeordnet, das einen Ölstand OIL_H des in dem Messrohr 30 befindlichen Öls erfasst. Das Sensorelement 36 ist beispielsweise ein resistives Messelement. Die Pumpe pumpt das Öl aus der Ölwanne 12 während sich die Brennkraftmaschine außerhalb eines Betriebszustands des Motorstops befindet, das heißt, wenn die Kurbelwelle 8 sich dreht pumpt die Pumpe Öl aus der Ölwanne durch den Ölkreislauf. Das aus dem Ölkreislauf zurückfließende Öl tropft zurück in die Ölwanne 12. Wenn die Brennkraftmaschine still steht, läuft ein Großteil des Öls aus dem Ölkreislauf zurück in die Ölwanne 12 und sammelt sich in dieser. Es kann ferner vorteilhaft sein, wenn in der Ölwanne 12 in der Nähe des Ölstandsensors 28 Fließhindernisse 38 ausgebildet sind, die beispielsweise Schwappbleche sein können. Durch die Fließhindernisse 38 kann das Ausfließverhalten des Öls aus dem Messrohr 30 beeinflusst werden.

In der Steuereinrichtung 14 ist ein Programm zum Ermitteln einer Größe, die charakteristisch ist für die Viskosität des Öls, gespeichert und wird während des Betriebs der Brennkraftmaschine abgearbeitet.

Das Programm wird in einem Schritt S1 gestartet, in dem Variablen initialisiert werden, insbesondere wird in dem Schritt S1 ein Zähler n mit dem Wert Null initialisiert.

In einem Schritt S2 wird ermittelt, ob vorgegebene Betriebsbedingungen BB vorliegen. Die vorgegebenen Betriebsbedingungen BB können vorliegen, wenn ein Teil oder auch alle der nachfolgend aufgeführten Bedingungen erfüllt werden, je nach Ausführungsform des Programms. Dabei können diese Bedingungen insbesondere darauf Einfluss haben, wie lange die vorgegebenen Betriebsbedingungen BB erfüllt sind. Es wird so beispielsweise geprüft, wie groß eine Stillstandszeitdauer T_S ist. Eine Bedingung, die abhängt von der Stillstandszeitdauer T_S kann erfüllt sein, wenn die Stillstandszeitdauer T_S einen vorgegebenen Schwellenwert für die Stillstandszeitdauer T_S überschreitet, so zum Beispiel zehn Minuten. Sie kann jedoch auch ein Maß dafür sein, wie lange die vorgegebenen Betriebsbedingungen maximal erfüllt sein können. Dadurch kann beispielsweise erreicht werden, dass die vorgegebenen Betriebsbedingungen BB nur direkt nach einem Start der Brennkraftmaschine für eine vorgebbare Zeitdauer erfüllt sind.

Ferner wird eine weitere Bedingung abhängig von der Öltemperatur TOIL, der Umgebungstemperatur TAMB und/oder der Kühlmitteltemperatur TCO ermittelt. Da die Viskosität des Öls sehr stark abhängt von der Temperatur des Öls in dem Messrohr 30 kommt dem Verlauf der Temperatur des Öls in dem Messrohr zum Ermitteln der Größe, die charakteristisch ist für die Viskosität des Öls, eine hohe Bedeutung zu. Bevorzugt wird die Bedingung abhängig von der Öltemperatur TOIL und/oder der Umgebungstemperatur TAMB und/oder der Kühlmitteltemperatur TCO als erfüllt angesehen, wenn sich diese Temperaturen bei aufeinander folgenden Durchläufen der Schritte S2 weniger als ein vorgebbarer Schwellenwert für diese Temperaturen ändern. Bevorzugt wird aus der Öltemperatur TOIL und/oder der Umgebungstemperatur TAMB und/oder der Kühlmitteltemperatur TCO ein Schätzwert der Öltemperatur in dem Messrohr 30 ermittelt und dieser dann mit dem Schwellenwert verglichen.

Darüber hinaus kann als weitere Bedingung für das Erfülltsein der vorgegebene Betriebsbedingungen BB auch noch vorgesehen sein, zu prüfen, welchen Verlauf eine Drehzahl und/oder eine Fahrzeuggeschwindigkeit V_FZG und/oder eine Beschleunigung A_FZG hat. Die vorgegebenen Betriebsbedingungen sollten bei einer hohen Dynamik der Drehzahl und/oder der Fahrzeuggeschwindigkeit V_FZG und/oder der Beschleunigung A_FZG nicht erfüllt sein, da in diesem Fall ein präzises Ermitteln der Größe, die charakteristisch ist für die Viskosität des Öls nur sehr schwer möglich ist.

Ferner kann eine weitere Bedingung abhängig von einer zeitlichen Veränderlichkeit GRAD_OIL des Ölstandes OIL_H ermittelt werden. Diese Bedingung sollte lediglich dann erfüllt sein, wenn bevorzugt die zeitliche Veränderlichkeit GRAD OIL des Ölstandes OIL_H einen monoton fallenden Verlauf aufweist.

Durch das Erfülltsein der vorgegebenen Betriebsbedingungen BB wird ein so genanntes Messfenster festgelegt, während dessen der Ölstand OIL_H erfasst wird zum Ermitteln der zeitlichen Veränderlichkeit GRAD_OIL des Ölstandes OIL_H. Insbesondere bei geeignet niedrigen Temperaturen des Öls in dem Messrohr 30, von zum Beispiel 20°C hat dieses Messfenster dann eine für ein präzises Ermitteln der Größe, die charakteristisch ist für die Viskosität des Öls, günstige Breite.

In einem Schritt S3 wird anschließend geprüft, ob die vorgegebene Betriebsbedingung erfüllt ist, was beispielsweise bei dem Wert TRUE der Fall ist. Ist die Bedingung des Schrittes S3 erfüllt, so wird in einem Schritt S4 ein Zähler n um den Wert eins inkrementiert.

Anschließend wird in einem Schritt S5 der aktuelle Ölstand OIL_H[n] und der zugehörige aktuelle Zeitpunkt t[n] erfasst. Anschließend verharrt das Programm für eine vorgegebene Wartezeitdauer T_W in einem Schritt S6, bevor die Bedingung des Schrittes S2 erneut geprüft wird.

Ist die Bedingung des Schrittes S3 hingegen nicht erfüllt, so wird in einem Schritt S7 geprüft, ob der Zähler n größer ist als eins. Ist dies nicht der Fall, so kann keine zeitliche Veränderlichkeit GRAD_OIL des Ölstandes OIL_H ermittelt werden und der Zähler n wird in einem Schritt S8 auf den Wert Null zurückgesetzt. Anschließend an den Schritt S8 wird die Bearbeitung des Programms in dem Schritt S6 fortgesetzt.

Ist die Bedingung des Schrittes S7 hingegen erfüllt, so wird in einem Schritt S10 die zeitliche Veränderlichkeit GRAD OIL des Ölstandes OIL_H abhängig von den in den vorangegangenen Durchläufen des Schrittes S5 ermittelten Wertepaaren des Ölstandes OIL_H und der zugeordneten Zeitpunkte t ermittelt. Dies kann beispielsweise durch Differenzbildung jeweils zweier zeitlich benachbarter Werte des Ölstandes OIL_H und der zugeordneten Zeitpunkte und eine entsprechende Division der so ermittelten Differenzen erfolgen. Bevorzugt werden die dann so ermittelten zeitlichen Veränderlichkeiten GRAD_OIL des Ölstandes OIL_H im Folgenden anschließend noch gemittelt. Alternativ kann jedoch die zeitliche Veränderlichkeit GRAD_OIL auch beispielsweise lediglich abhängig von einem bei einem erstmaligen Erfülltsein des Schrittes S3 ermittelten Wertepaar des Ölstandes OIL_H und der zugeordneten Zeit t und dem bei dem letztmaligen Erfülltsein der Bedingung des Schrittes S3 ermittelten Wertepaar des Ölstandes OIL_H und der Zeit t durch entsprechende Differenzbildung und Division der Differenzen ermittelt werden. Die zeitliche Veränderlichkeit GRAD_OIL, die in dem Schritt S10 ermittelt wurde, kann dann die Größe darstellen, die charakteristisch ist für die Viskosität des Öls. In diesem Fall wird dann die Bearbeitung im Anschluss an den Schritt S10 erneut in dem Schritt S8 fortgesetzt.

Bevorzugt werden davor jedoch noch einer oder mehrere der folgenden Schritte abgearbeitet. So wird beispielsweise im Anschluss an den Schritt S10 ein Schritt S13 abgearbeitet, in dem eine Viskositätskennzahl VISC_KZ abhängig von zumindest der zeitlichen Veränderlichkeit GRAD_OIL und einer Referenz REF_GRAD des Ölstandes OIL_H ermittelt wird. Die Referenz REF_GRAD kann fest vorgegeben sein, sie kann jedoch auch abhängig von der bei der Erfassung der der Ermittlung der zeitlichen Veränderlichkeit GRAD_OIL zugrunde liegenden Messwerte vorliegenden Temperatur des Öls in dem Messrohr 30 ermittelt werden. Charakteristisch für die Temperatur des Öls in dem Messrohr 30 sind eine oder mehrere Größen der Öltemperatur TOIL, der Umgebungstemperatur TAMB und der Kühlmitteltemperatur TCO.

Darüber hinaus kann das Ermitteln der Viskositätskennzahl VISC_KZ auch abhängig von dem Wert des Zählers n erfolgen, der ein Maß dafür ist, für wie viele aufeinander folgende Durchläufe des Schrittes S3 dessen Bedingung erfüllt war, was ein Maß für Güte der ermittelten zeitlichen Veränderlichkeit GRAD_OIL des Ölstandes OIL_H sein kann.

Alternativ oder zusätzlich wird in einem Schritt S15 ein Viskositätswert VISC abhängig von der zeitlichen Veränderlichkeit GRAD_OIL des Ölstands OIL_H, der Umgebungstemperatur TAMB und/oder der Öltemperatur TOIL und/oder der Kühlmitteltemperatur TCO und/oder der Stillstandszeitdauer T_S und/oder des Standes des Zählers n und gegebenenfalls weiterer Größen ermittelt. Dies kann über entsprechende Kennfelder oder auch ein sonstiges Modell für die Viskosität erfolgen.

Alternativ oder zusätzlich kann in einem Schritt S17 eine Viskositätsänderung D_VISC abhängig von zumindest der zeitliche Veränderlichkeit GRAD_OIL des Ölstandes OIL_H und/oder dem Stand des Zählers n und/oder weiterer Größen, wie beispielsweise der des Schrittes S15, ermittelt werden.

Abhängig von der zeitlichen Veränderlichkeit GRAD_OIL und/oder der Viskositätskennzahl VISC_KZ und/oder dem Viskositätswert VISC und/oder der Viskositätsänderung D_VISC wird beispielsweise im Folgenden ermittelt, ob ein Wechsel des in der Brennkraftmaschine befindlichen Öls notwendig ist und abhängig von dem Ergebnis dieser Prüfung erfolgt dann bevorzugt eine Anzeige des empfohlenen Ölwechsels über ein Display eines Bordcomputers des Fahrzeugs.

Bei einem möglichst gleichbleibenden Verlauf der Öltemperatur TOIL und/oder der Umgebungstemperatur TAMB und/oder der Kühlmitteltemperatur TCO, während die Bedingung des Schrittes S3 bei aufeinander folgenden Durchläufen des Schrittes S3 erfüllt ist, kann gewährleistet werden, dass lediglich eine vernachlässigbare Temperaturzu- oder -abnahme des außerhalb des Messrohres befindlichen Öles während der nachgeordneten Durchläufe der Schritte S5 und der diesen zugeordneten Erfassen des Ölstandes OIL_H stattfindet.

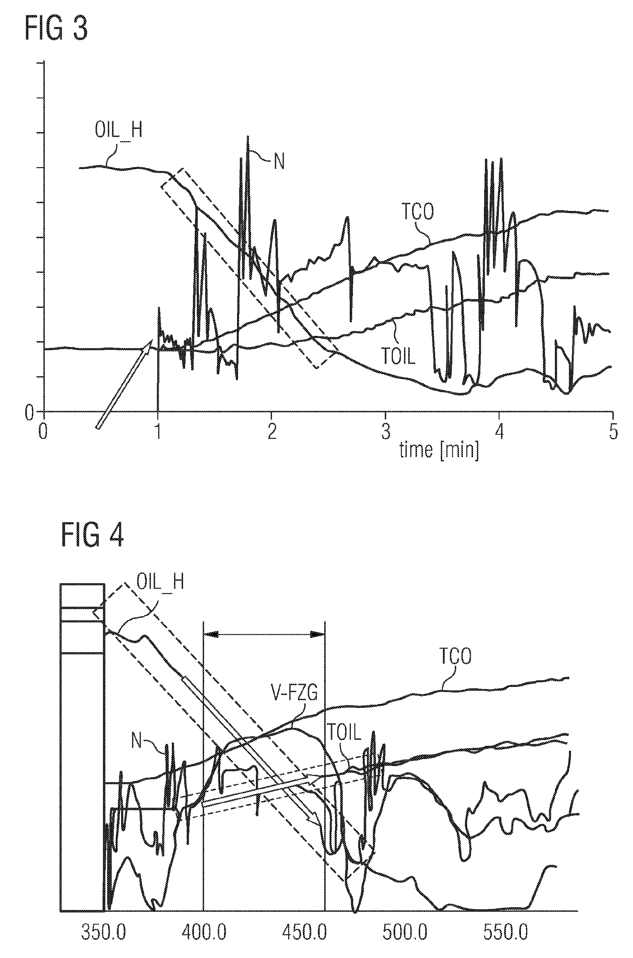

Anhand der Figuren 3 und 4 sind die zeitlichen Verläufe verschiedener Größen dargestellt. Auf eine Skalierung der Ordinate wurde verzichtet, da nur eine qualitative Aussage anhand der Verläufe der Größen erfolgen soll und die Größen verschiedenste Einheiten haben. In Figur 3 sind ausgehend von einem Start der Brennkraftmaschine der Ölstand OIL_H mit einem Ausgangswert von in etwa 70 mm, eine Öltemperatur TOIL, die zum Start der Brennkraftmaschine in etwa 20°C beträgt, die Kühlmitteltemperatur TCO die zum Start ebenfalls in etwa 20°C beträgt, und die Drehzahl N aufgetragen. Das gestrichelt eingezeichnete Rechteck bezeichnet einen möglichen Bereich, in dem die vorgegebenen Betriebsbedingungen BB erfüllt sein können.

Bei dem in Figur 4 dargestellten Verlauf hat die Öltemperatur TOIL beim Start der Brennkraftmaschine in etwa einen Wert von 5°C gleiches gilt in etwa für die Kühlmitteltemperatur TCO. Der Ölstand OIL_H beträgt hier beim Start der Brennkraftmaschine in etwa 48 mm. Ferner sind in der Figur 4 die Verläufe der Drehzahl N und der Fahrzeuggeschwindigkeit V_FZG aufgetragen. Auch hier bezeichnet wieder der rechteckig gestrichelte Bereich einen möglichen zeitlichen Bereich, in dem die vorgegebenen Betriebsbedingungen BB erfüllt sein können, bevorzugt sind die vorgegebenen Betriebsbedingungen BB jedoch erfüllt, in dem durch den Doppelpfeil dargestellten zeitlichen Bereich.

## Patentansprüche

1. Verfahren zum Ermitteln einer Größe, die charakteristisch ist für die Viskosität eines Öls einer Brennkraftmaschine mit einem Ölstandsensor (28), der in einer Ölwanne (12) angeordnet ist und der ein Messrohr (30) hat, das über mindestens eine Ausnehmung (32, 34) mit dem das Messrohr (30) umgebenden Öl kommuniziert, bei dem
- geprüft wird, ob vorgegebene Betriebsbedingungen (BB) vorliegen,
- wenn die vorgegebenen Betriebsbedingungen (BB) vorliegen, eine zeitliche Veränderlichkeit(GRAD_OIL) des Ölstands (OIL_H) in dem Messrohr (30) ermittelt wird, und
- abhängig von der zeitlichen Veränderlichkeit (GRAD_OIL) des Ölstandes (OIL_H) die Größe ermittelt wird, die charakteristisch ist für die Viskosität des Öls.

2. Verfahren nach Anspruch 1, bei dem die vorgegebenen Betriebsbedingungen (BB) abhängen von einer Stillstandszeitdauer (T_S) der Brennkraftmaschine.

3. Verfahren nach einem der vorstehenden Ansprüche, bei dem die vorgegebenen Betriebsbedingungen (BB) abhängen von einer erfassten Öltemperatur (TOIL) und/oder einer Umgebungstemperatur (TAMB) der Brennkraftmaschine und/oder einer Kühlmitteltemperatur (TCO).

4. Verfahren nach Anspruch 3, bei dem die vorgegebenen Betriebsbedingungen (BB) nur dann erfüllt sein können, wenn die erfasste Temperatur (TOIL) und/oder die Umgebungstemperatur (TAMB) und/oder die Kühlmitteltemperatur (TCO) sich weniger als ein vorgegebener Schwellenwert ändern.

5. Verfahren nach einem der vorstehenden Ansprüche, bei dem die vorgegebenen Betriebsbedingungen (BB) abhängen von einer Drehzahl (N) einer Kurbelwelle (8) der Brennkraftmaschine und/oder einer Fahrgeschwindigkeit (V_FZG) eines Kraftfahrzeugs, in dem die Brennkraftmaschine angeordnet ist und/oder einer Beschleunigung (A_FZG) des Kraftfahrzeugs.

6. Verfahren nach einem der vorstehenden Ansprüche, bei dem die vorgegebenen Betriebsbedingungen (BB) davon abhängen, ob die zeitliche Veränderlichkeit (GRAD_OIL) des Ölstands (OIL_H) im Wesentlichen monoton fällt.

7. Verfahren nach einem der vorstehenden Ansprüche, bei dem die zeitliche Veränderlichkeit (GRAD_OIL) des Ölstands (OIL_H) während des Vorliegens der vorgegebenen Betriebsbedingungen (BB) gemittelt wird.

8. Verfahren nach einem der vorstehenden Ansprüche, bei dem die zeitliche Veränderlichkeit (GRAD_OIL) des Ölstands (OIL_H) verglichen wird mit einer Referenz (REF_GRAD) und abhängig von dem Vergleich die Größe ermittelt wird, die charakteristisch ist für die Viskosität des Öls.

9. Verfahren nach einem der vorstehenden Ansprüche, bei dem die Größe, die charakteristisch ist für die Viskosität, abhängig davon ermittelt wird, für welche Zeitdauer (t) die vorgegebenen Betriebsbedingungen (BB) durchgehend vorliegen.

10. Verfahren nach einem der vorstehenden Ansprüche, bei dem die Größe, die charakteristisch ist für die Viskosität des Öls, die Viskosität ist und die Viskosität abhängig von der zeitlichen Veränderlichkeit (GRAD_OIL) des Ölstands (OIL_H) und der erfassten Öltemperatur (TOIL) und/oder der Umgebungstemperatur (TAMB) und/oder der Kühlmitteltemperatur (TCO) ermittelt wird.

11. Verfahren nach einem der Ansprüche 1 bis 9, bei dem die Größe, die charakteristisch ist für die Viskosität des Öls, die zeitliche Veränderlichkeit (GRAD_OIL) des Ölstands (OIL_H) ist.

12. Verfahren nach einem der Ansprüche 1 bis 9, bei dem die Größe, die charakteristisch ist für die Viskosität, eine Viskositätsänderung (D_VISC) ist.
